# FASCICULE DE BREVET EUROPEEN

(11) **EP 3 361 947 B1**
(45) Date de publication et mention de la délivrance du brevet: **06.05.2020**
(21) Numéro de dépôt: 15794216.0
(22) Date de dépôt: 14.10.2015
(51) Int. Cl.: A61B 5/087, A61B 5/097, A61B 5/00

(54) **APPAREIL DE MESURE DES RESPIRATIONS BUCCALES ET NASALES D'UN ÊTRE HUMAIN**
VORRICHTUNG ZUR MESSUNG DER MUND- UND NASENATMUNG EINES MENSCHEN
APPARATUS FOR MEASURING THE ORAL AND NASAL BREATHING OF A HUMAN BEING

(43) Date de publication de la demande: 22.08.2018
(73) Titulaire: Aerophonoscope, 22210 Plemet (FR)
(72) Inventeur: GUILLERM, Gérard, F-22210 Plemet (FR)
(74) Mandataire: Regimbeau
(86) Numéro de dépôt international: PCT/FR2015/052769
(87) Numéro de publication internationale: WO 2016/128627

(56) Documents cités:
- WO-A1-2015/131219
- US-A- 5 311 875
- US-A1- 2006 276 718
- US-A1- 2008 038 207
- US-A1- 2008 161 709

## Description

L'invention concerne un appareil de diagnostic et/ou de rééducation des respirations buccales et nasales d'un être humain.

Le domaine d'application de l'invention concerne les appareils médicaux utilisés dans le domaine médical, notamment en orthophonie et/ou en orthodontie.

L'une des exigences de l'appareil dans ce domaine est qu'il doit pouvoir s'adapter à différents types d'examen, selon le diagnostic à faire, ou à différentes morphologies de patients, par exemple selon leur âge.

Ainsi, un orthophoniste peut souhaiter que l'appareil détecte des anomalies telles que par exemple la respiration buccale trop importante au détriment de la respiration nasale, des asymétries de la perméabilité narinaire, ou que de la rééducation de la respiration nasale puisse être faite à l'aide de l'appareil.

Un orthophoniste peut également souhaiter évaluer les capacités fonctionnelles du voile du palais, notamment que l'appareil détecte l'encombrement du rhinopharynx par des végétations adénoïdes, ou la bonne position du massif lingual, ou les capacités contractiles du voile.

Un orthophoniste peut également souhaiter évaluer par l'appareil de mesure une insuffisance vélopharyngée (IVP). Le mécanisme vélopharyngé d'un être humain est chargé de diriger la transmission de l'énergie acoustique et la pression de l'air à la fois dans la cavité buccale et la /ou au moins une deuxième tête amovible. Lorsque ce mécanisme est altéré d'une certaine façon, une condition appelée insuffisance vélo-pharyngée peut se développer.

Un orthodontiste peut souhaiter évaluer par l'appareil les effets orthopédiques d'une expansion maxillaire provoquée par un appareil d'expansion.

Le document US-A-2006/0276718 concerne un appareil de détection selon le préambule de la revendication 1, comportant des canules placées dans les narines et la bouche d'une personne, reliées à une unité de logement contenant un capteur de mesure d'un signal de flux respiratoire de la personne pendant son sommeil, et une unité de traitement pour stocker des données temporelles du signal de flux respiratoire, pour diagnostiquer des troubles du sommeil de la personne à partir de ces données.

Le document US-A-5 311 875 concerne un capteur de respiration, comportant un transducteur ayant un film en PVDF à propriétés piézoélectriques et pyroélectriques et des électrodes, qui définissent une zone sensible aux variations de température des gaz respiratoires passant sur des lobes de ce film devant être disposés sous les narines et devant la bouche d'une personne, pour surveiller les apnées du sommeil de la personne à partir de la tension de sortie du transducteur.

Le document US-A-2008/0038207 concerne un dispositif pour mesurer les particules exhalées par une personne.

Le document US 2008/0161709 concerne un appareil de surveillance de respiration, comportant une pièce devant être placée devant la bouche d'une personne, la pièce étant connectée d'une manière détachable à une entrée de fluide et incluant un dispositif de mesure de débit massique pour mesurer le volume d'air inhalé par la personne, la pièce incluant une valve permettant à l'air exhalé par la personne d'entrer dans l'entrée de fluide, cette valve incluant un masque flexible agencé pour être disposé sur la bouche de la personne.

Le document WO 2015/131219 A1 est aussi important pour comprendre le contexte de l'invention.

L'invention vise à obtenir un appareil de diagnostic et/ou de rééducation des respirations buccales et nasales d'un être humain, qui permette de remplir les exigences ci-dessus pour différents types de diagnostics et/ou différents types de patients.

L'invention a pour objet un appareil de diagnostic et/ou de rééducation des respirations buccales et nasales d'un être humain suivant la revendication 1.

Suivant un mode de réalisation de l'invention, l'appareil comporte comme tête amovible un jeu de plusieurs têtes amovibles, pouvant être montées chacune d'une manière interchangeable dans la position de solidarisation sur le manche de préhension.

Suivant un mode de réalisation de l'invention, l'appareil comporte au moins une première tête amovible agencée pour un diagnostic d'orthodontie et/ou au moins une deuxième tête amovible agencée pour un diagnostic d'orthophonie.

Suivant un mode de réalisation de l'invention, l'appareil comporte plusieurs têtes amovibles de tailles différentes.

Suivant un mode de réalisation de l'invention, deux ouvertures supérieures sont prévues, destinées à être positionnées respectivement sous les deux narines de l'être humain.

Suivant un mode de réalisation de l'invention, les premiers moyens de montage comportent une cavité ouverte, les deuxièmes moyens de montage comportent une partie saillante, apte à être insérée dans la cavité dans une direction d'insertion pour se trouver dans la position de solidarisation.

Suivant un mode de réalisation de l'invention, la partie saillante et la cavité sont agencées, de telle sorte que, dans la position de solidarisation de la tête amovible sur le manche de préhension, la partie saillante du manche se trouve dans la cavité de la tête pour y être bloquée et empêcher une rotation de la tête par rapport au manche autour de la direction d'insertion.

Suivant un mode de réalisation de l'invention, la partie saillante comporte au moins une surface extérieure et la cavité comporte au moins une surface intérieure, la surface extérieure et la surface intérieure étant agencées pour se trouver l'une contre l'autre en position de solidarisation pour empêcher une rotation de la tête par rapport au manche autour de la direction d'insertion.

Suivant un mode de réalisation de l'invention, la surface extérieure et la surface intérieure sont planes.

Suivant un mode de réalisation de l'invention, la surface extérieure et la surface intérieure sont parallèles à la direction d'insertion.

Suivant un mode de réalisation de l'invention, les premiers moyens de connexion comportent une première interface de connexion, située dans un fond de la cavité, les deuxièmes moyens comportent une deuxième interface de connexion, située à une extrémité supérieure de la partie saillante, la deuxième interface de connexion étant connectée à la première interface de connexion lorsque la partie saillante est insérée dans la cavité dans la direction d'insertion pour se trouver dans la position de solidarisation.

Suivant un mode de réalisation de l'invention, il est prévu au moins un capteur de mesure de présence de flux d'air en face de chaque ouverture supérieure et au moins un autre capteur de mesure de présence de flux d'air en face de chaque ouverture arrière.

Suivant un mode de réalisation de l'invention, il est prévu comme capteur de présence de flux d'air au moins un conducteur nu de thermistance, s'étendant entre deux extrémités reliées à un circuit électrique faisant passer un courant de chauffage dans le conducteur nu de thermistance,

le conducteur nu de thermistance provoquant, en réponse au flux d'air passant sur lui, une variation de la résistance électrique du conducteur nu de thermistance entre ses extrémités, apte à être détectée par des moyens de détection du circuit électrique.

Suivant un mode de réalisation de l'invention, le circuit électrique comporte des moyens de polarisation pour imposer un courant électrique prescrit constant dans le conducteur nu de thermistance et des moyens de détection de la tension électrique présente entre les deux extrémités du conducteur nu de thermistance.

Suivant un mode de réalisation de l'invention, le circuit électrique comporte des moyens de polarisation pour imposer une tension électrique prescrite constante entre les deux extrémités du conducteur nu de thermistance et des moyens de détection du courant électrique passant dans le conducteur nu de thermistance.

Suivant un mode de réalisation de l'invention, il est prévu comme capteur de présence de flux d'air au moins un capteur de son, positionné en face de l'ouverture arrière.

Suivant un mode de réalisation de l'invention, l'appareil comporte une pièce de filtration des flux d'air, amovible et destinée à être fixée contre la première paroi extérieure.

L'invention sera mieux comprise en référence aux modes de réalisation décrits ci-dessous aux dessins annexés donnés à titre d'exemples non limitatifs, dessins sur lesquels :

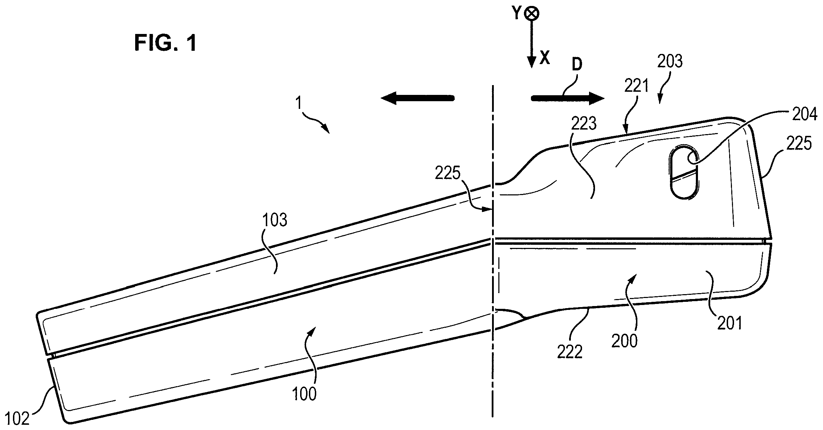

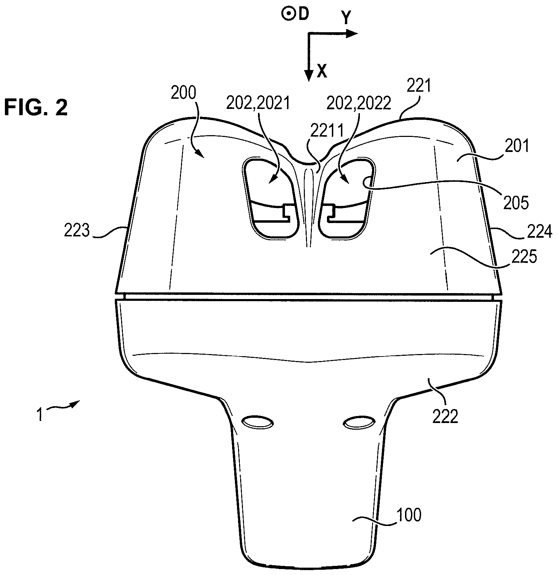

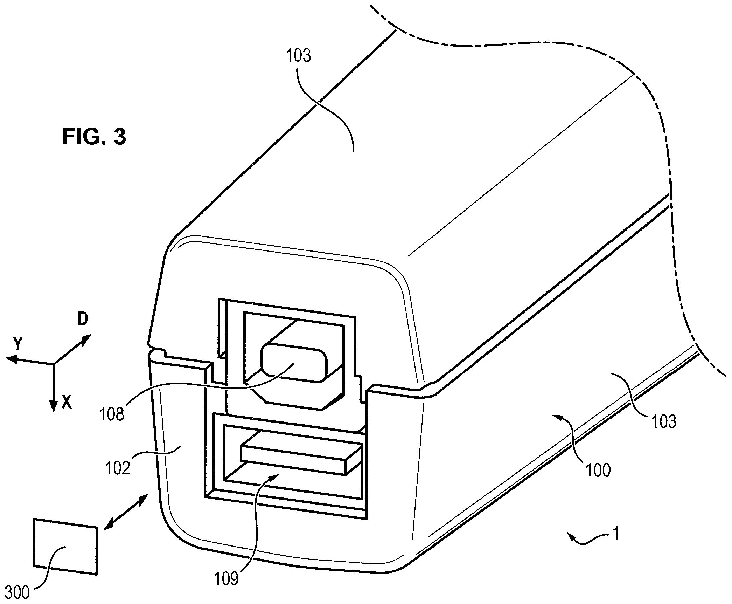

- les figures 1, 2 et 3 sont des vues schématiques de côté, de dessus et de dessous de l'appareil suivant un mode de réalisation de l'invention,

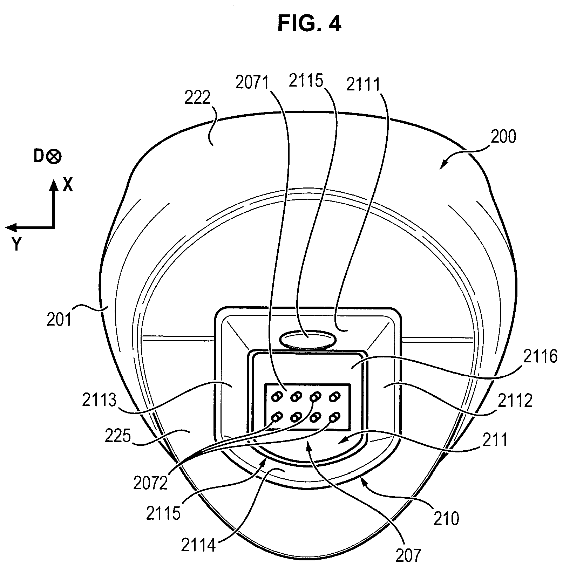

- la figure 4 est une vue schématique de dessous de la tête de l'appareil à l'état désolidarisé par rapport au manche suivant un mode de réalisation de l'invention,

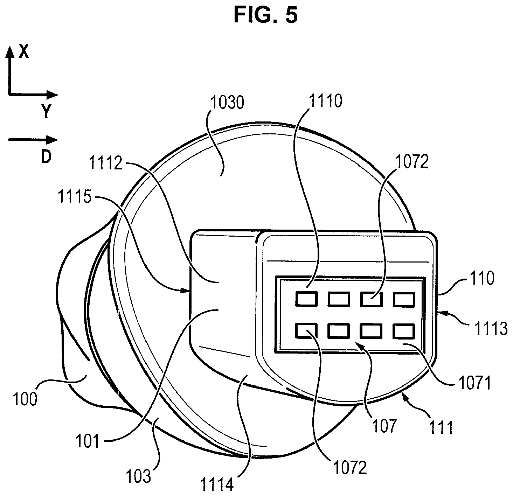

- la figure 5 est une vue schématique en perspective de dessus du manche de l'appareil à l'état désolidarisé par rapport à la tête suivant un mode de réalisation de l'invention,

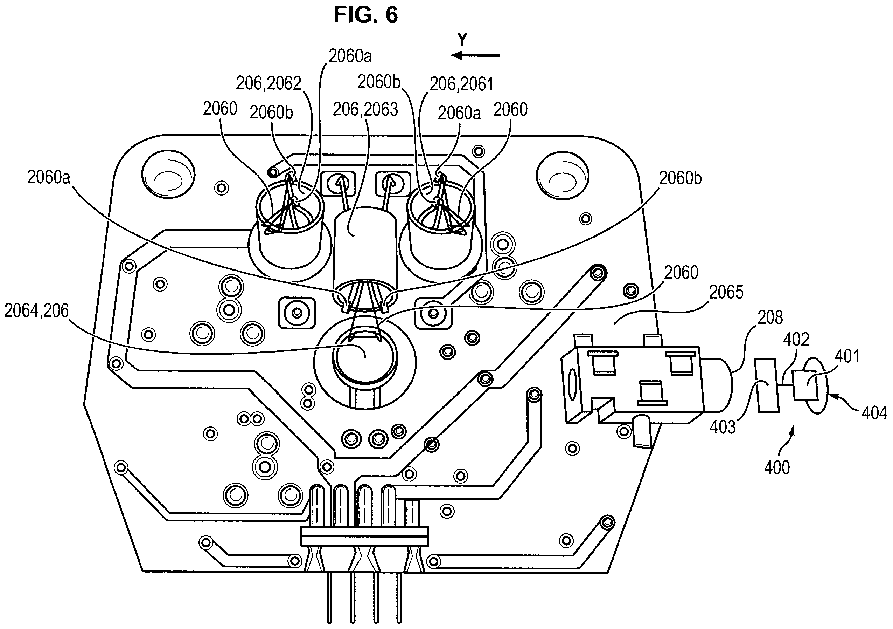

- la figure 6 est une vue schématique de capteurs situés dans la tête de l'appareil suivant un mode de réalisation de l'invention,

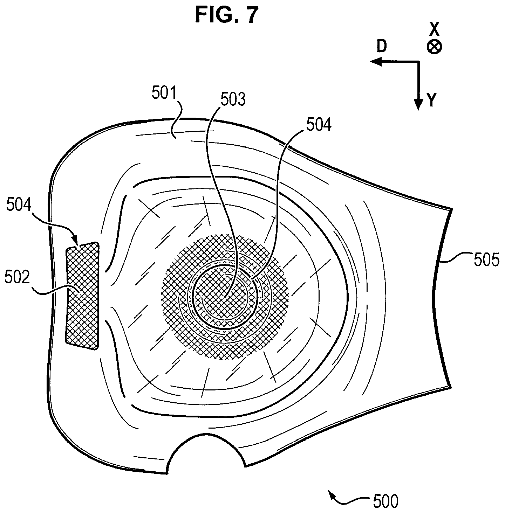

- la figure 7 est une vue schématique d'une pièce amovible de filtration d'air de l'appareil suivant un mode de réalisation de l'invention,

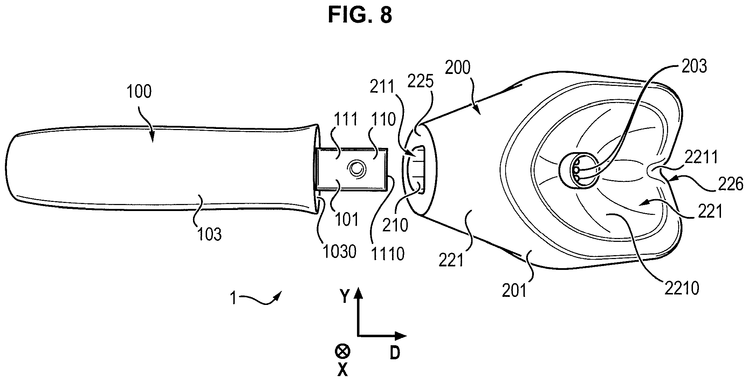

- la figure 8 est une vue schématique de l'arrière de l'appareil suivant un mode de réalisation de l'invention, dans lequel le manche est à l'état désolidarisé par rapport à la tête,

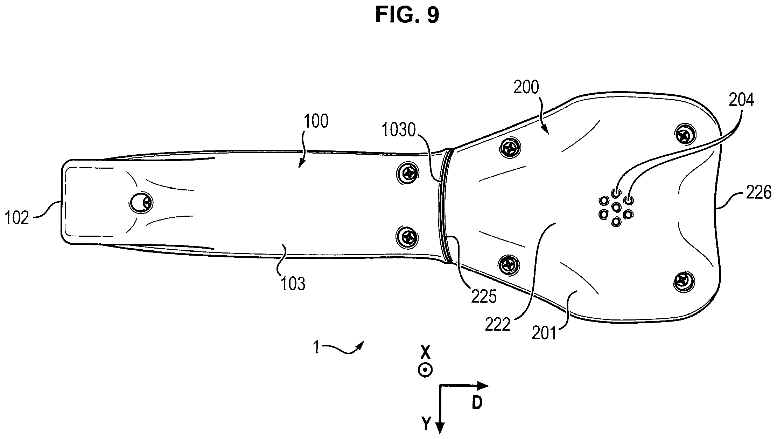

- la figure 9 est une vue schématique de face de l'appareil suivant un mode de réalisation de l'invention, dans lequel le manche est dans la position de solidarisation par rapport à la tête.

Aux figures sont représentés des modes de réalisation d'un appareil 1 de diagnostic et/ou de rééducation de la respiration buccale et de la respiration nasale d'un être humain.

L'appareil 1 de diagnostic et/ou de rééducation de la respiration tel que décrit comporte deux parties :
- un manche 100 de préhension,
- une tête 200.

Suivant un mode de réalisation, la tête 200 peut être montée et enlevée sur le manche 100 et est donc amovible. La tête 200 comporte au moins un capteur 206 de mesure de présence de flux d'air, pour fournir une mesure de la respiration buccale et/ou nasale. Chaque capteur 206 de mesure de présence de flux d'air est apte à fournir au moins un signal de mesure de présence de flux d'air. Chaque capteur 206 de mesure est apte à détecter la présence d'un flux d'air ou à mesurer une grandeur représentative de ce flux d'air, par exemple sa vitesse. Il peut être prévu un seul ou plusieurs capteur(s) 206 de mesure de présence de flux d'air.

La tête 200 comporte une première paroi extérieure 201 dans laquelle se trouvent au moins une ouverture supérieure ou nasale 202 et au moins une ouverture arrière ou buccale 203. L'ouverture supérieure 202 est destinée à être positionnée sous les narines de l'être humain. L'ouverture arrière 203 est destinée à être positionnée devant la bouche de l'être humain. Il peut également être prévu dans la paroi 201 une ou plusieurs autres ouvertures 204 d'échappement et/ou d'aspiration d'air.

La paroi 201 délimite un premier compartiment 205 dans la tête 200 et est munie de moyens de positionnement, dans le premier compartiment 205, du ou des capteur(s) 206 de mesure de présence ou de flux d'air.

Ainsi, la tête 200 forme une unité autonome comportant le ou les capteur(s) 206 de mesure de respiration.

Suivant un mode de réalisation, il est prévu comme ouverture supérieure 202 une première ouverture supérieure 2021 et une deuxième ouverture supérieure 2022, distincte de la première ouverture supérieure 2021. Les deux ouvertures supérieures 2021, 2022 sont côte à côte, respectivement à droite et à gauche, dans la direction transversale latérale et horizontale Y (orientée de la droite vers la gauche) et sont destinées à être positionnées respectivement sous les deux narines droite et gauche de l'être humain. Suivant un mode de réalisation, il est prévu au moins un capteur 2061, 2062 de mesure de présence de flux d'air en face respectivement de chaque ouverture supérieure 2021, 2022. Il est donc prévu dans ce cas le capteur 2061 pour la mesure de présence de flux d'air sous la narine gauche et le capteur 2062 pour la mesure de présence de flux d'air sous la narine gauche.

Suivant un mode de réalisation, il est prévu au moins un autre capteur 206, 2063 de mesure de présence de flux d'air en face de chaque ouverture arrière 203.

La paroi 201 comporte une surface arrière 221 destinée à être positionnée contre la lèvre supérieure et/ou contre la bouche de l'être humain, et une surface avant 222, éloignée de la face arrière 221 selon une direction X et raccordée à la face arrière 221 par deux surfaces latérales droite et gauche 223 et 224 et par une surface inférieure 225 et une surface supérieure 226, éloignée de la surface inférieure 225 selon une direction D. La direction D est une direction verticale allant de bas en haut. La ou les ouvertures 202, 2021 et 2022 se trouvent dans la surface supérieure 226. La ou les ouvertures 203 se trouve dans la surface arrière 221. La surface arrière 221 comporte un renfoncement 2210 ouvert vers l'arrière autour de la ou des ouvertures arrière 203, afin que l'utilisateur puisse positionner sa bouche dans ce renfoncement 2210. Une échancrure 2211 s'étend en haut de la surface arrière 221 et va du renfoncement 2210 à la surface supérieure 226. L'échancrure 2211 sert à loger dedans l'arête inférieure du nez.

Ainsi, le premier capteur 2061 de mesure de présence de flux d'air mesure la présence d'un flux d'air provenant de la première ouverture supérieure 2021 ou allant vers la première ouverture supérieure 2021, selon que l'être humain souffle ou aspire de l'air par sa narine droite, lorsque celle-ci est positionnée en face de la première ouverture supérieure 2021.

Ainsi, le deuxième capteur 2062 de mesure de présence de flux d'air mesure la présence d'un flux d'air provenant de la deuxième ouverture supérieure 2022 ou allant vers la deuxième ouverture supérieure 2022, selon que l'être humain souffle ou aspire de l'air par sa narine gauche, lorsque celle-ci est positionnée en face de la deuxième ouverture supérieure 2022.

Ainsi, le troisième capteur 2063 de mesure de présence de flux d'air mesure la présence d'un flux d'air provenant de l'ouverture arrière 203 ou allant vers l'ouverture arrière 203, selon que l'être humain souffle ou aspire de l'air par sa bouche, lorsque celle-ci est positionnée en face de l'ouverture arrière 203.

Suivant un mode de réalisation, la tête amovible 200 comporte des premiers moyens 210 de montage aptes à coopérer avec des deuxièmes moyens 110 de montage prévus sur une partie d'extrémité 101 du manche 100 de préhension. Les premiers moyens 210 de montage sont séparés des deuxièmes moyens 110 de montage. Les premiers moyens 210 de montage sont aptes à coopérer avec les deuxièmes moyens 110 de montage dans une position de solidarisation de la tête amovible 200 sur le manche 100 de préhension. Les premiers moyens 210 de montage sont aptes à être désolidarisés par rapport aux deuxièmes moyens 110 de montage pour permettre d'enlever la tête amovible 200 par rapport au manche 100 de préhension.

Par exemple, dans le mode de réalisation représenté aux figures, les premiers moyens 210 de montage sont du type femelle et comportent une cavité 211 ouverte, tandis que les deuxièmes 110 de montage sont du type mâle et comportant une partie saillante 111. La partie saillante 111 est apte à être insérée dans la cavité 211 dans la direction D d'insertion pour se trouver dans la position de solidarisation. La partie saillante 111 et la cavité 211 sont agencées, de telle sorte que, dans la position de solidarisation de la tête amovible 200 sur le manche 100 de préhension, la partie saillante 111 du manche 100 se trouve dans la cavité 211 de la tête 200 pour y être bloquée et empêcher une rotation de la tête 200 par rapport au manche 100 autour de la direction D d'insertion. Il y a par exemple une complémentarité de forme de la partie saillante 111 et de la cavité 211 pour réaliser ce blocage en rotation. A cet effet, par exemple, le contour extérieur de la partie saillante 111 autour de la direction D d'insertion de la partie saillante 111 dans la cavité 211 correspond au contour intérieur de la cavité 211 autour de cette direction D. Ces contours sont par exemple autres que circulaires. A cet effet, le contour extérieur de la partie saillante 111 autour de la direction D d'insertion de la partie saillante 111 dans la cavité 211 et le contour intérieur de la cavité 211 autour de cette direction D comportent par exemple chacun une ou plusieurs surfaces planes, qui se trouvent l'une contre l'autre dans la position de solidarisation. Ainsi, la forme de la partie saillante 111 et de la cavité 211 permet une seule position de solidarisation entre le manche 100 et la tête 200 et assure un détrompage lors du montage de la tête 200 sur le manche 100.

Les moyens de montage 110, 210 peuvent comporter des moyens de blocage dans la position de solidarisation, tels que par exemple une saillie 2115 coopérant avec un renfoncement 1117 transversalement à la direction D d'insertion. dans un mode de réalisation, la cavité 211 peut comporter la saillie 2115 s'étendant transversalement à la direction D d'insertion entre son ouverture inférieure 2115 et son fond 2116 (par exemple sur la surface 2111), cette saillie 2115 étant apte à entrer, dans la position de solidarisation, dans le renfoncement ou évidement 1117 de la partie saillante 111, situé entre ses extrémités inférieure 1115 et supérieure 1110 (par exemple dans la surface 1111), pour bloquer la tête 100 dans cette position de solidarisation sur le manche 100. La saillie 2115 est apte à sortir du renfoncement 1117 par traction sur le manche 200 dans le sens inverse de la direction D d'insertion par rapport à la tête 100 pour les éloigner l'un de l'autre. La saillie 2115 est apte à entrer et à sortir du renfoncement 1117 par élasticité du matériau formant la saillie 2115 et/ou du matériau formant le renfoncement 1117. Bien entendu, la saillie 2115 pourrait être prévue sur la partie saillante 111 et le renfoncement pourrait être prévu dans la cavité 211.

La cavité ouverte 211 est délimitée selon la direction D entre une ouverture inférieure 2115, par laquelle peut être insérée la partie saillante 111, et un fond 2116. L'ouverture inférieure 2115 de la cavité 211 se trouve dans la surface inférieure 225 de la tête 200.

Dans le mode de réalisation représenté aux figures, le contour extérieur de la partie saillante 111 et le contour intérieur de la cavité 211 comportent une ou plusieurs surfaces planes, parallèle(s) à la direction D d'insertion. Par exemple, la partie saillante 111 comporte une première surface extérieure plane 1111 raccordée à ses extrémités à une deuxième surface extérieure plane 1112 et à une troisième surface extérieure plane 1113, elle-même raccordées, à distance de la surface 1111, à une quatrième surface extérieure courbe 1114. La surface extérieure 103 du manche 100 est raccordée à la partie saillante 111 par un épaulement intérieur 1030 entourant l'extrémité inférieure 1115 de la partie saillante 111. Les surfaces 1111, 1112, 1113 et 1114 sont parallèles à la direction D d'insertion de la partie saillante 111 dans la cavité 211. Les moyens 210 de montage comportent, pour délimiter la cavité 211, une première surface intérieure plane 2111, raccordée à ses extrémités à une deuxième surface intérieure plane 2112 et à une troisième surface intérieure plane 2113, elle-même raccordées, à distance de la première surface intérieure plane 2111, à une quatrième surface intérieure courbe 2114. Les surfaces 1112 et 1113 sont éloignées l'une de l'autre et sont, par exemple, chacune perpendiculaires à la surface 1111. Les surfaces 2112 et 2113 sont éloignées l'une de l'autre et sont, par exemple, chacune perpendiculaires à la surface 2111.

Par exemple, la dimension transversale de la première surface extérieure 1111 correspond ou est égale à la dimension transversale de la première surface intérieure 2111, la dimension transversale de la deuxième surface extérieure 1112 correspond ou est égale à la dimension transversale de la première surface intérieure 2112, la dimension transversale de la troisième surface extérieure 1113 correspond ou est égale à la dimension transversale de la troisième surface intérieure 2113, et la dimension transversale de la quatrième surface extérieure 1114 correspond ou est égale à la dimension transversale de la quatrième surface intérieure 2114. La dimension transversale est prise dans un plan perpendiculaire à la direction D d'insertion de la partie saillante 111 dans la cavité 211. Ainsi, dans la position de solidarisation, les surfaces 1111, 1112, 1113 et 1114 se trouvent respectivement contre les surfaces 2111, 2112, 2113 et 2114. Dans la position de solidarisation, la surface 225 se trouve contre l'épaulement 1030. La direction X est par exemple perpendiculaire à la direction Y. La direction D est par exemple perpendiculaire à la direction Y et à la direction X.

Dans un mode de réalisation, la tête amovible 200 comporte des premiers moyens 207 de connexion du ou des capteur(s) 206. Ces premiers moyens 207 de connexion sont configurés pour être connectés, dans la position de solidarisation, à des deuxièmes moyens 107 de connexion prévus sur la partie d'extrémité 101 du manche 100 de préhension. Dans la position de solidarisation, les premiers moyens 207 de connexion sont donc connectés aux deuxièmes moyens 107 de connexion pour transmettre aux deuxièmes moyens 107 de connexion le signal de mesure de présence de flux d'air, provenant du ou des capteur(s).

Dans un mode de réalisation, les premiers moyens 207 de connexion comportent une première interface 2071 de connexion et les deuxièmes moyens 107 comportent une deuxième interface 1071 de connexion. Dans la position de solidarisation, la deuxième interface 1071 de connexion est connectée à la première interface 2071 de connexion. Par exemple, la première interface 2071 de connexion est située dans les premiers moyens 210 de montage, notamment dans la cavité 211, et la deuxième interface 1071 de connexion est située dans les deuxièmes moyens 110 de montage, notamment dans la partie saillante 111.

Par exemple, l'interface 2071 se trouve dans le fond 1116 de la cavité 211 et la deuxième interface 1071 se trouve à l'extrémité supérieure 1110 de la partie saillante 111.

Par exemple, l'interface 2071 est du type connecteur mâle comportant plusieurs conducteurs 2072 faisant saillie à l'encontre de la direction D d'insertion, tandis que l'interface 1071 est du type connecteur femelle et comporte plusieurs trous 1072 servant à la réception des conducteurs 2072 dans la position de solidarisation. Dans la partie saillante 111, les deuxièmes moyens 107 de connexion comportent d'autres conducteurs positionnés dans les trous 1072 de telle sorte que, dans la position de solidarisation, les conducteurs 2072 de la tête 200 soient en contact électrique avec ces autres conducteurs des moyens 107 de connexion.

Ainsi, dans un mode de réalisation, la coopération des premiers moyens 207 de connexion avec les deuxièmes moyens 107 de connexion contribue au blocage de la tête 200 sur le manche 100 dans la position de solidarisation.

Dans un mode de réalisation, les deuxièmes moyens 107 de connexion sont connectés à une unité 300 de traitement du signal de mesure, cette unité 300 étant située à l'extérieur du manche 100 de préhension et à l'extérieur de la tête 200.

Par exemple, le manche 100 comporte, en une deuxième partie d'extrémité inférieure 102, une ou plusieurs autres interface(s) 108, 109 servant à la connexion des deuxièmes moyens 107 de connexion à l'unité 300 de traitement. L'interface 109 est par exemple du type USB, permettant d'enficher dessus une prise USB d'un câble USB, dont l'autre extrémité pourra être connectée à l'unité 300. La première partie 101 d'extrémité supérieure est éloignée de la deuxième partie d'extrémité inférieure 102 dans la direction D d'insertion.

La ou les autres interface(s) 108, 109 sont connectées par des conducteurs électriques non représentés, et/ou par un module d'un circuit électrique intermédiaire, aux conducteurs de l'interface 1072, positionnés dans la première extrémité supérieure 1110 du manche 100.

Dans un mode de réalisation, le manche 100 de préhension comporte, autour de la direction D d'insertion, une surface extérieure 103 servant à la préhension par la main de l'être humain, dont les narines et la bouche sont positionnées en face respectivement des ouvertures 202, 203 de la tête 200.

Suivant un mode de réalisation, le capteur 206 de présence de flux d'air est du type thermistance. Par exemple, le capteur 206 comporte au moins un conducteur électrique nu 2060 de thermistance, qui est positionné dans le compartiment 205 pour être exposé au flux d'air provenant de l'ouverture associée 202 ou 203 ou allant vers l'ouverture associée 202 ou 203. Ce conducteur nu 2060 de thermistance a entre ses deux extrémités 2060a et 2060b une résistance électrique, qui varie en fonction de la température. Le capteur 206 de présence comporte en outre un circuit électrique 2065 relié aux deux extrémités 2060a et 2060b du conducteur nu 2060 de thermistance, pour faire passer dans ce conducteur nu 2060 de thermistance un courant de chauffage de celui-ci. Par conséquent, le conducteur nu 2060 de thermistance provoque, en réponse au flux d'air passant sur lui, une variation de la résistance électrique du conducteur 2060 entre ses extrémités 2060a et 2060b. Le circuit électrique 2065 comporte des moyens de détection de la variation de résistance électrique du conducteur nu 2060 de thermistance.

Par exemple, ce circuit électrique 2065 comporte des moyens de polarisation pour imposer un courant électrique prescrit constant dans le conducteur nu 2060 de thermistance, et des moyens de détection de la tension électrique présente entre les extrémités 2060a et 2060b du conducteur nu 2060 de thermistance, en réponse au flux d'air passant sur lui. Le flux d'air passant sur le conducteur nu 2060 de thermistance le refroidit plus ou moins et fait donc varier sa résistance. L'abaissement de la température du conducteur nu 2060 de thermistance augmente sa résistance. Par conséquent, plus la quantité de flux d'air passant sur le conducteur nu 2060 de thermistance est grande, plus la tension présente entre les extrémités 2060a et 2060b de ce conducteur 2060 est grande. Les moyens de détection sont prévus pour mesurer la tension électrique présente entre les extrémités 2060a et 2060b du conducteur nu 2060 de thermistance, pour le courant prescrit constant imposé par les moyens de polarisation présents dans le circuit 2065.

Bien entendu, il pourrait être prévu, au lieu de cela, que le circuit 2065 comporte des moyens de polarisation pour imposer une tension électrique prescrite constante entre les extrémités 2060a et 2060b du conducteur nu 2060 de thermistance et des moyens de détection de la variation du courant électrique passant dans le conducteur nu 2060a et 2060b de thermistance, en réponse au flux d'air passant sur lui.

Dans un mode de réalisation, le conducteur nu 2060 de thermistance du capteur 206 est formé par un fil métallique ou un filament métallique. Ce fil métallique ou filament métallique est par exemple à incandescence et peut être par exemple du type de ceux utilisés dans des ampoules d'éclairage par fil ou filament incandescent. Toutefois, la tension électrique appliquée au conducteur nu 2060 de thermistance est une basse tension, inférieure à 220 V et par exemple inférieure à 50 V. Ce conducteur nu 2060 de thermistance peut donc être sélectionné, suivant un mode de réalisation, parmi les fils ou filaments des ampoules d'éclairage par incandescence, dont on a retiré la capsule extérieure transparente ou translucide (habituellement en verre), afin de laisser le fil ou filament directement en contact avec le flux d'air. Le conducteur nu 2060 de thermistance du capteur 206 est par exemple formé par un filament métallique oxydé au préalable, afin que celui-ci soit partiellement corrodé et devienne par la suite inoxydable. Par exemple, pour ce faire, le filament métallique a été brûlé au préalable, par exemple sur un support de test, au moins 20 minutes. Le conducteur nu 2060 de thermistance a ainsi une bonne résistance à l'oxydation.

Dans un mode de réalisation, il est prévu comme capteur 206 de présence de flux d'air au moins un quatrième capteur 2064 de son, positionné en face de l'ouverture arrière 203. Ce capteur 2064 peut être prévu en plus du ou des capteur(s) 2061, 2062, 2063.

Le ou les capteur(s) 206 permettent de mesurer la présence d'un flux d'air venant ou allant vers une narine, les deux narines ou la bouche de l'être humain, et donc de détecter chacune les respirations buccales et nasales de cet être humain.

Ainsi, l'appareil 1 permet de déterminer si l'être humain respire par la bouche ou par le nez, ou par les deux, s'il existe une asymétrie de la respiration nasale entre la narine droite et la narine gauche. L'unité 300 de traitement du signal de mesure comporte un écran de visualisation du ou des signaux de mesure du ou des capteur(s) 206, pour visualiser l'évolution temporelle de ce signal de mesure.

Le fait que la tête 200 soit amovible sur le manche 100 permet de changer la tête, par exemple, lorsqu'elle est défectueuse. Il peut être prévu deux têtes amovibles identiques 200, ce qui permet d'avoir en plus de la tête utilisée une tête de remplacement.

Suivant un mode de réalisation, l'appareil 1 comporte comme tête amovible 200 un jeu de plusieurs têtes amovibles 200, pouvant être montées chacune d'une manière interchangeable dans la position de solidarisation sur le manche 100 de préhension. Ainsi, il est possible de prévoir plusieurs têtes amovibles 200 de tailles différentes. Les têtes de tailles différentes permettent de s'adapter à différentes morphologies d'êtres humains ou à des personnes d'âges différents. Il pourra ainsi être prévu une tête 200 de taille plus petite pour un jeune enfant, par exemple d'âge inférieur ou égal à cinq ans, une deuxième tête amovible 200 pour un enfant d'âge de plus de cinq ans jusqu'à dix ans, de taille plus grande que la première tête amovible 200, une troisième tête amovible 200 pour un enfant de plus de dix ans jusqu'à quinze ans, de taille supérieure à celle de la deuxième tête amovible, et une quatrième tête amovible 200 pour un être humain de plus de quinze ans et/ou un adulte, cette quatrième tête étant d'une taille supérieure à celle de la troisième tête.

Il peut également être prévu au moins une première tête amovible agencée pour un diagnostic d'orthodontie et/ou au moins une deuxième tête amovible agencée pour un diagnostic d'orthophonie.

Suivant un mode de réalisation, il est prévu dans la première paroi 201 de la tête 200, par exemple dans sa surface latérale 223 ou 224, une autre interface 208 reliée au circuit électrique 2065 se trouvant dans le compartiment 205. Cette interface 208 est prévue pour être connectée à un élément 400, qui est amovible par rapport à la tête 200. Cet élément amovible 400 comporte un autre capteur 401 de vibrations du larynx de l'être humain et des moyens de connexion 402 de ce capteur 401 à une autre interface 403 de connexion de cet élément 400. L'interface 403 de l'élément 400 est apte à être connectée à l'interface 208 de la tête 200. Ce capteur 401 de son comporte par exemple au moins un élément piézoélectrique pour transformer une force provoquée par les vibrations du larynx de l'être humain en une tension électrique, laquelle est détectée par des moyens de détection du circuit électrique 2065. L'élément 400 peut comporter en outre un moyen 404 de maintien du capteur 401 contre le larynx de l'être humain, ce moyen de maintien 404 comportant par exemple un élastique passé autour du cou de l'être humain pour plaquer le capteur 401 entre l'élastique et le larynx.

Les premiers moyens 207 de connexion et les deuxièmes moyens 107 de connexion servent également à alimenter en électricité les capteurs 206 et/ou 301 pour leur fonctionnement, l'électricité étant fournie via l'interface 108 et/ou 109 par un moyen d'alimentation électrique prévu dans l'unité 300.

Dans un mode de réalisation, il peut être prévu une pièce 500 d'hygiène amovible ou de protection ou de filtration des flux d'air, amovible et destinée à être fixée contre la première paroi extérieure 201 de la tête 200. La pièce 500 laisse passer les flux d'air et est destinée à être fixée contre la face arrière 221 et/ou la face supérieure 226 de la tête et/ou autour de la tête contre ses faces 221, 222, 223, 224 et 226 au moins en partie ou entièrement. Cette pièce 500 sert à protéger les capteurs et les ouvertures 202, 203 de l'humidité ou des particules solides provenant de la respiration de l'être humain. Cette pièce 500 peut comporter un ou plusieurs filtres 502, 503 du flux d'air, pour retenir les particules liquides et/ou solides provenant de l'être humain. Lorsque la pièce 500 est en position de montage sur la tête 200, le filtre 502 nasal est positionné en face de la ou des ouvertures 202, 2021, 2022 et le filtre 503 est positionné en face de l'ouverture arrière 203, afin d'empêcher le passage de particules liquides et/ou solides vers ces ouvertures 202, 203. La pièce 500 d'hygiène comporte par exemple une coque 501 en un matériau souple ou élastique, pour pouvoir être enfilée et enlevée sur la tête 200. Le filtre 502 est fixé dans une ouverture supérieure 504 de la coque 501. Le filtre 503 est fixé dans une ouverture arrière 504 de la coque 501. La coquille comporte en outre un bord inférieur permettant le passage de la surface inférieure 225 et/ou un bord avant 506 permettant le passage de la face avant 222. La coque 501 a par exemple la même forme que la partie de la tête 200 qu'elle protège. La pièce 500 peut être une partie consommable de l'appareil 1 et peut être jetée et remplacée par une autre pièce après usage de l'appareil 1.

L'appareil 1 suivant l'invention permet ainsi au praticien d'effectuer une étude simultanée et comparative des flux aériens nasaux et/ou oraux. L'appareil 1 suivant l'invention permet d'établir un bilan clinique de la ventilation aérienne supérieure et des capacités fonctionnelles du voile du palais. Ainsi, l'appareil 1 suivant l'invention permet d'évaluer les insuffisances vélaires par localisation et appréciation des déperditions nasales. L'appareil 1 suivant l'invention permet d'évaluer de manière précise et reproductible les troubles respiratoires et de faire la rééducation de la respiration et de la phonation. Les différentes têtes 200 permettent de s'adapter à des morphologies différentes du nez et de la bouche (enfant, adulte, forme de visage). Etant donné que 95 % des pannes viendront d'un capteur 206, il ne faut changer dans ce cas, grâce à la tête amovible, que la tête 200.

L'observation par le praticien (par exemple orthodontiste ou orthophoniste) des courbes temporelles des signaux de mesure sur l'unité 300 de traitement permet d'effectuer un diagnostic sur la respiration de l'être humain. L'appareil 1 permet également d'analyser la respiration buccale et/ou nasale de l'être humain lorsqu'il prononce une parole. La détection par l'appareil 1 d'un flux d'air nasal lors de la répétition de phonèmes, de mots ou de phrases prononcés par l'être humain, objective le phénomène de la déperdition nasale et en quantifie la sévérité.

Grâce à la sensibilité du ou des capteur(s) 206 de flux d'air nasal, les mouvements d'ouverture et de fermeture du sphincter vélo-pharyngé sont clairement visibles sur les courbes proposées par l'unité 300, objectivant le potentiel musculaire du sphincter et permettant de vérifier :
- sa rigidité,
- ses possibilités d'ouverture et de fermeture complète,
- sa rapidité d'exécution des mouvements d'ouverture et de fermeture,
- sa stabilité.

L'analyse de la parole avec l'appareil 1 permet ainsi de déterminer l'origine étiologique de l'IVP selon trois profils typiques :
- le profil dysmorphique (organique, structurel) : béances vélopharyngées (voile ou néo-voile court avant ou après opération et/ou cavum profond) ; voile rigide (constitutionnel ou post opératoire), voile cicatriciel,
- le profil dysfonctionnel : voile potentiellement performant, mais qui a un retard ou une insuffisance de développement (cas de retard mental, surdité, névrose, immaturité psychologique, mimétisme culturel) ou qui conserve des habitudes post chirurgicales (hypotonie fonctionnelle du voile),
- le profil neurologique : neuropathies flasques ou spasmodiques, syndromes dystoniques ou dyspraxiques généraux ou localisés à la sphère bucco-faciale.

Bien entendu, les modes de réalisation et exemples décrits peuvent être combinés l'un avec l'autre ou être sélectionnés indépendamment l'un de l'autre.

De toute façon, l'invention est défini par les revendications attachées.

## Revendications

1. Appareil (1) de diagnostic et/ou de rééducation des respirations buccales et nasales d'un être humain,
l'appareil (1) comportant au moins un capteur (206) de mesure de présence de flux d'air, apte à fournir au moins un signal de mesure de présence de flux d'air, et une tête amovible (200) ayant une première paroi extérieure (201) munie d'au moins une ouverture supérieure (202), destinée à être positionnée sous les narines de l'être humain, d'au moins une ouverture arrière (203) destinée à être positionnée devant la bouche de l'être humain,
la première paroi extérieure (201) délimite un premier compartiment (205) et est munie de moyens de positionnement, dans le premier compartiment (205), du capteur (206) de mesure de présence de flux d'air,
l'appareil comportant en outre un manche (100) de préhension manuelle, pour positionner l'ouverture supérieure (202) de la tête amovible (200) sous les narines de l'être humain et l'ouverture arrière (203) de la tête amovible (200) devant la bouche de l'être humain, lorsque la tête amovible (200) se trouve dans une position de solidarisation sur le manche (100) de préhension,
la tête amovible (200) ayant des premiers moyens (210) de montage aptes, dans la position de solidarisation de la tête amovible (200) sur le manche (100) de préhension, à coopérer avec des deuxièmes moyens (110) de montage prévus sur une partie d'extrémité (101) du manche (100) de préhension, les premiers moyens (210) de montage pouvant être désolidarisés des deuxièmes moyens (110) de montage pour permettre d'enlever la tête amovible (200) par rapport au manche (100) de préhension,
la tête amovible (200) comportant des premiers moyens (207) de connexion du capteur (206), configurés pour être connectés, dans la position de solidarisation, à des deuxièmes moyens (107) de connexion prévus sur la partie d'extrémité (101) du manche (100) de préhension pour transmettre le signal de mesure de présence de flux d'air aux deuxièmes moyens (107) de connexion,
les deuxièmes moyens (107) de connexion étant connectés à une unité (300) de traitement du signal de mesure, située à l'extérieur du manche (100) de préhension, l'unité (300) de traitement comportant un écran de visualisation pour visualiser l'évolution temporelle du signal de mesure.

2. Appareil suivant la revendication 1, **caractérisé en ce que** l'appareil comporte comme tête amovible un jeu de plusieurs têtes amovibles (200), pouvant être montées chacune d'une manière interchangeable dans la position de solidarisation sur le manche de préhension.

3. Appareil suivant la revendication 2, **caractérisé en ce qu'**il comporte au moins une première tête amovible (200) agencée pour un diagnostic d'orthodontie et/ou au moins une deuxième tête amovible (200) agencée pour un diagnostic d'orthophonie.

4. Appareil suivant la revendication 2 ou 3, **caractérisé en ce qu'**il comporte plusieurs têtes amovibles (200) de tailles différentes.

5. Appareil suivant l'une quelconque des revendications précédentes, **caractérisé en ce que** deux ouvertures supérieures (2021, 2022) sont prévues, destinées à être positionnées respectivement sous les deux narines de l'être humain.

6. Appareil suivant l'une quelconque des revendications précédentes, **caractérisé en ce que** les premiers moyens (210) de montage comportent une cavité (211) ouverte, les deuxièmes moyens (110) de montage comportent une partie saillante (111), apte à être insérée dans la cavité (211) dans une direction (D) d'insertion pour se trouver dans la position de solidarisation.

7. Appareil suivant la revendication 6, **caractérisé en ce que** la partie saillante (111) et la cavité (211) sont agencées, de telle sorte que, dans la position de solidarisation de la tête amovible (200) sur le manche (100) de préhension, la partie saillante (111) du manche (100) se trouve dans la cavité (211) de la tête (200) pour y être bloquée et empêcher une rotation de la tête (200) par rapport au manche (100) autour de la direction (D) d'insertion.

8. Appareil suivant la revendication 7, **caractérisé en ce que** la partie saillante (111) comporte au moins une surface extérieure (2111, 2112, 2113) et la cavité (211) comporte au moins une surface intérieure (1111, 1112, 1113), la surface extérieure (2111, 2112, 2113) et la surface intérieure (1111, 1112, 1113) étant agencées pour se trouver l'une contre l'autre en position de solidarisation pour empêcher une rotation de la tête (200) par rapport au manche (100) autour de la direction (D) d'insertion.

9. Appareil suivant la revendication 8, **caractérisé en ce que** la surface extérieure (2111, 2112, 2113) et la surface intérieure (1111, 1112, 1113) sont planes.

10. Appareil suivant la revendication 8 ou 9, **caractérisé en ce que** la surface extérieure (2111, 2112, 2113) et la surface intérieure (1111, 1112, 1113) sont parallèles à la direction (D) d'insertion.

11. Appareil suivant l'une quelconque des revendications 6 à 10, **caractérisé en ce que** les premiers moyens (207) de connexion comportent une première interface (2071) de connexion, située dans un fond (1116) de la cavité (211), les deuxièmes moyens (107) comportent une deuxième interface (1071) de connexion, située à une extrémité supérieure (1110) de la partie saillante (111), la deuxième interface (1071) de connexion étant connectée à la première interface (2071) de connexion lorsque la partie saillante (111) est insérée dans la cavité (211) dans la direction (D) d'insertion pour se trouver dans la position de solidarisation.

12. Appareil suivant l'une quelconque des revendications précédentes, **caractérisé en ce qu'**il est prévu au moins un capteur (2061, 2062) de mesure de présence de flux d'air en face de chaque ouverture supérieure (202, 2021, 2022) et au moins un autre capteur (2063) de mesure de présence de flux d'air en face de chaque ouverture arrière (203).

13. Appareil suivant l'une quelconque des revendications précédentes, **caractérisé en ce qu'**il est prévu comme capteur (206) de présence de flux d'air au moins un conducteur nu (2060) de thermistance, s'étendant entre deux extrémités reliées à un circuit électrique (2065) faisant passer un courant de chauffage dans le conducteur nu (2060) de thermistance,
le conducteur nu (2060) de thermistance provoquant, en réponse au flux d'air passant sur lui, une variation de la résistance électrique du conducteur nu (2060) de thermistance entre ses extrémités (2060a, 2060b), apte à être détectée par des moyens de détection du circuit électrique (2065).

14. Appareil suivant la revendication 13, **caractérisé en ce que** le circuit électrique (2065) comporte des moyens de polarisation pour imposer un courant électrique prescrit constant dans le conducteur nu (2060) de thermistance et des moyens de détection de la tension électrique présente entre les deux extrémités (2060a, 2060b) du conducteur nu (2060) de thermistance.

15. Appareil suivant la revendication 13, **caractérisé en ce que** le circuit électrique (2065) comporte des moyens de polarisation pour imposer une tension électrique prescrite constante entre les deux extrémités (2060a, 2060b) du conducteur nu (2060) de thermistance et des moyens de détection du courant électrique passant dans le conducteur nu (2060) de thermistance.

16. Appareil suivant l'une quelconque des revendications précédentes, **caractérisé en ce qu'**il est prévu comme capteur (206) de présence de flux d'air au moins un capteur (2064) de son, positionné en face de l'ouverture arrière (203).

17. Appareil suivant l'une quelconque des revendications précédentes, **caractérisé en ce qu'**il comporte une pièce (500) de filtration des flux d'air, amovible et destinée à être fixée contre la première paroi extérieure (201).

## Patentansprüche

1. Vorrichtung (1) zur Messung der Mund- und Nasenatmung eines Menschen,
wobei die Vorrichtung (1) mindestens einen Sensor (206) zur Messung der Anwesenheit eines Luftstroms, der imstande ist, mindestens ein Messsignal der Anwesenheit eines Luftstroms bereitzustellen, und einen lösbaren Kopf (200) aufweist, der eine erste Außenwand (201) aufweist, die mit mindestens einer oberen Öffnung (202), die zur Positionierung unter den Nasenlöchern des Menschen bestimmt ist, und mit mindestens einer hinteren Öffnung (203), die zur Positionierung vor dem Mund des Menschen bestimmt ist, ausgestattet ist,
wobei die erste Außenwand (201) ein erstes Abteil (205) begrenzt und mit Positionierungsmitteln, im ersten Abteil (205), des Sensors (206) zur Messung der Anwesenheit eines Luftstroms ausgestattet ist,
wobei die Vorrichtung ferner einen Handgriff (100) aufweist, um die obere Öffnung (202) des lösbaren Kopfs (200) unter die Nasenlöcher des Menschen und die hintere Öffnung (203) des lösbaren Kopfs (200) vor dem Mund des Menschen zu positionieren, wenn sich der lösbare Kopf (200) in einer festen Verbindungsposition auf dem Griff (100) befindet,
wobei der lösbare Kopf (200) erste Montagemittel (210) hat, die in der festen Verbindungsposition des lösbaren Kopfs (200) auf dem Griff (100) imstande sind, mit zweiten Montagemitteln (110) zusammenzuwirken, die auf einem Endabschnitt (101) des Griffs (100) vorgesehen sind, wobei die ersten Montagemittel (210) von den zweiten Montagemitteln (110) lösbar sind, um das Abnehmen des lösbaren Kopfs (200) in Bezug auf den Griff (100) zu erlauben,
wobei der lösbare Kopf (200) erste Verbindungsmittel (207) des Sensors (206) aufweist, die konfiguriert sind, um in der festen Verbindungsposition mit zweiten Verbindungsmitteln (107) verbunden zu sein, die auf dem Endabschnitt (101) des Griffs (100) vorgesehen sind, um das Signal zur Messung der Anwesenheit eines Luftstroms an die zweiten Verbindungsmittel (107) zu übertragen,
wobei die zweiten Verbindungsmittel (107) mit einer Verarbeitungseinheit (300) des Messsignals verbunden sind, die sich außerhalb des Griffs (100) befindet, wobei die Verarbeitungseinheit (300) einen Anzeigebildschirm aufweist, um die zeitliche Entwicklung des Messsignals anzuzeigen.

2. Vorrichtung nach Anspruch 1, **dadurch gekennzeichnet, dass** die Vorrichtung als lösbaren Kopf einen Satz mehrerer lösbarer Köpfe (200) aufweist, die jeweils untereinander austauschbar in der festen Verbindungsposition auf dem Griff anbringbar sind.

3. Vorrichtung nach Anspruch 2, **dadurch gekennzeichnet, dass** sie mindestens einen ersten lösbaren Kopf (200) aufweist, der für eine Orthodontiediagnose eingerichtet ist, und/oder mindestens einen zweiten lösbaren Kopf (200), der für eine Orthophoniediagnose eingerichtet ist.

4. Vorrichtung nach Anspruch 2 oder 3, **dadurch gekennzeichnet, dass** sie mehrere lösbare Köpfe (200) unterschiedlicher Größen aufweist.

5. Vorrichtung nach einem der vorangehenden Ansprüche, **dadurch gekennzeichnet, dass** zwei obere Öffnungen (2021, 2022) vorgesehen sind, die jeweils zur Positionierung unter den zwei Nasenlöchern des Menschen bestimmt sind.

6. Vorrichtung nach einem der vorangehenden Ansprüche, **dadurch gekennzeichnet, dass** die ersten Montagemittel (210) einen offenen Hohlraum (211) aufweisen, wobei die zweiten Montagemittel (110) einen hervorstehenden Abschnitt (111) aufweisen, der in den Hohlraum (211) in einer Einsetzrichtung (D) einsetzbar ist, um sich in der festen Verbindungsposition zu befinden.

7. Vorrichtung nach Anspruch 6, **dadurch gekennzeichnet, dass** der hervorstehende Abschnitt (111) und der Hohlraum (211) derart eingerichtet sind, dass in der festen Verbindungsposition des lösbaren Kopfs (200) auf dem Griff (100) sich der hervorstehende Abschnitt (111) des Griffs (100) im Hohlraum (211) des Kopfs (200) befindet, um dort blockiert zu sein und eine Rotation des Kopfs (200) in Bezug auf den Griff (100) um die Einsetzrichtung (D) zu verhindern.

8. Vorrichtung nach Anspruch 7, **dadurch gekennzeichnet, dass** der hervorstehende Abschnitt (111) mindestens eine äußere Fläche (2111, 2112, 2113) aufweist und der Hohlraum (211) mindestens eine innere Fläche (1111, 1112, 1113) aufweist, wobei die äußere Fläche (2111, 2112, 2113) und die innere Fläche (1111, 1112, 1113) einrichtet sind, um sich gegeneinander in fester Verbindungsposition zu befinden, um eine Rotation des Kopfs (200) in Bezug auf den Griff (100) um die Einsetzrichtung (D) zu verhindern.

9. Vorrichtung nach Anspruch 8, **dadurch gekennzeichnet, dass** die äußere Fläche (2111, 2112, 2113) und die innere Fläche (1111, 1112, 1113) eben sind.

10. Vorrichtung nach Anspruch 8 oder 9, **dadurch gekennzeichnet, dass** die äußere Fläche (2111, 2112, 2113) und die innere Fläche (1111, 1112, 1113) zu der Einsetzrichtung (D) parallel sind.

11. Vorrichtung nach einem der Ansprüche 6 bis 10, **dadurch gekennzeichnet, dass** die ersten Verbindungsmittel (207) eine erste Verbindungsschnittstelle (2071) aufweisen, die sich in einem Boden (1116) des Hohlraums (211) befindet, wobei die zweiten Mittel (107) eine zweite Verbindungsschnittstelle (1071) aufweisen, die sich an einem oberen Ende (1110) des hervorstehenden Abschnitts (111) befindet, wobei die zweite Verbindungsschnittstelle (1071) mit der ersten Verbindungsschnittstelle (2071) verbunden ist, wenn der hervorstehende Abschnitt (111) in den Hohlraum (211) in der Einsetzrichtung (D) eingesetzt ist, um sich in der festen Verbindungsposition zu befinden.

12. Vorrichtung nach einem der vorangehenden Ansprüche, **dadurch gekennzeichnet, dass** mindestens ein Sensor (2061, 2062) zur Messung der Anwesenheit eines Luftstroms gegenüber jeder oberen Öffnung (202, 2021, 2022) und mindestens ein anderer Sensor (2063) zur Messung der Anwesenheit eines Luftstroms gegenüber jeder hinteren Öffnung (203) vorgesehen sind.

13. Vorrichtung nach einem der vorangehenden Ansprüche, **dadurch gekennzeichnet, dass** als Anwesenheitssensor (206) eines Luftstroms mindestens ein blanker Thermistorleiter (2060) vorgesehen ist, der sich zwischen zwei Enden erstreckt, die mit einem Stromkreis (2065) verbunden sind, der einen Heizstrom in den blanken Thermistorleiter (2060) fliessen lässt,
wobei der blanke Thermistorleiter (2060) als Antwort auf den Luftstrom, der auf ihm fliesst, eine Variation des elektrischen Widerstands des blanken Thermistorleiters (2060) zwischen seinen Enden (2060a, 2060b) bewirkt, die von Detektionsmitteln des Stromkreises (2065) detektierbar ist.

14. Vorrichtung nach Anspruch 13, **dadurch gekennzeichnet, dass** der Stromkreis (2065) Polarisationsmittel aufweist, um einen konstanten vorgeschriebenen elektrischen Strom in dem blanken Thermistorleiter (2060) zu erzwingen und Detektionsmittel der elektrischen Spannung, die zwischen den zwei Enden (2060a, 2060b) des blanken Thermistorleiters (2060) vorhanden ist.

15. Vorrichtung nach Anspruch 13, **dadurch gekennzeichnet, dass** der Stromkreis (2065) Polarisationsmittel aufweist, um eine konstante vorgeschriebene elektrische Spannung zwischen den zwei Enden (2060a, 2060b) des blanken Thermistorleiters (2060) zu erzwingen und Detektionsmittel des elektrischen Stroms, der in dem blanken Thermistorleiter (2060) fliesst.

16. Vorrichtung nach einem der vorangehenden Ansprüche, **dadurch gekennzeichnet, dass** als Anwesenheitssensor (206) eines Luftstroms mindestens ein Tonsensor (2064) vorgesehen ist, der gegenüber der hinteren Öffnung (203) positioniert ist.

17. Vorrichtung nach einem der vorangehenden Ansprüche, **dadurch gekennzeichnet, dass** sie ein Filterteil (500) zum Filtern der Luftströme aufweist, das lösbar ist und zur Befestigung an der ersten Außenwand (201) bestimmt ist.

## Claims

1. An apparatus (1) for diagnosing and/or rehabilitating the oral and nasal breathings of a human being,
the apparatus (1) including at least one sensor (206) for measuring the presence of air flow, capable of providing at least one air flow presence measurement signal, and a removable head (200) having a first outer wall (201) provided with at least one upper opening (202), intended to be positioned under the nostrils of the human being, at least one rear opening (203) intended to be positioned in front of the mouth of the human being,
the first outer wall (201) delimits a first compartment (205) and is provided with means for positioning, in the first compartment (205), the sensor (206) for measuring the presence of air flow,
the apparatus further including a manual gripping handle (100), for positioning the upper opening (202) of the removable head (200) under the nostrils of the human being and the rear opening (203) of the removable head (200) in front of the mouth of the human being, when the removable head (200) is in an engaged position on the gripping handle (100),
the removable head (200) having first mounting means (210) capable, in the engaged position of the removable head (200) on the gripping handle (100), of cooperating with second mounting means (110) provided on an end portion (101) of the gripping handle (100), the first mounting means (210) being able to be disengaged from the second mounting means (110) to allow the removable head (200) to be removed relative to the gripping handle (100),
the removable head (200) including first means (207) for connecting the sensor (206), configured to be connected, in the engaged position, to second connection means (107) provided on the end portion (101) of the gripping handle (100) for transmitting the air flow presence measurement signal to the second connection means (107),
the second connection means (107) being connected to a measurement signal processing unit (300), located outside the gripping handle (100), the processing unit (300) including a display screen for displaying the time evolution of the measurement signal.

2. The apparatus according to claim 1, **characterized in that** the apparatus includes as a removable head a set of several removable heads (200), each of which can be mounted in an interchangeable manner in the engaged position on the gripping handle.

3. The apparatus according to claim 2, **characterized in that** it includes at least one first removable head (200) arranged for a diagnosis of orthodontics and/or at least one second removable head (200) arranged for a diagnosis of speech therapy.

4. The apparatus according to claim 2 or 3, **characterized in that** it includes several removable heads (200) of different sizes.

5. The apparatus according to any one of the preceding claims, **characterized in that** two upper openings (2021, 2022) are provided, intended to be positioned respectively under the two nostrils of the human being.

6. The apparatus according to any one of the preceding claims, **characterized in that** the first mounting means (210) include an open cavity (211), the second mounting means (110) include a protruding portion (111), capable of being inserted into the cavity (211) in an insertion direction (D) to be in the engaged position.

7. The apparatus according to claim 6, **characterized in that** the protruding portion (111) and the cavity (211) are arranged, such that, in the engaged position of the removable head (200) on the gripping handle (100), the protruding portion (111) of the handle (100) is in the cavity (211) of the head (200) to be blocked therein and prevent rotation of the head (200) relative to the handle (100) about the insertion direction (D).

8. The apparatus according to claim 7, **characterized in that** the protruding portion (111) includes at least one outer surface (2111, 2112, 2113) and the cavity (211) includes at least one inner surface (1111, 1112, 1113), the outer surface (2111, 2112, 2113) and the inner surface (1111, 1112, 1113) being arranged to lie one against the other in the engaged position to prevent rotation of the head (200) relative to the handle (100) around the insertion direction (D).

9. The apparatus according to claim 8, **characterized in that** the outer surface (2111, 2112, 2113) and the inner surface (1111, 1112, 1113) are planar.

10. The apparatus according to claim 8 or 9, **characterized in that** the outer surface (2111, 2112, 2113) and the inner surface (1111, 1112, 1113) are parallel to the insertion direction (D).

11. The apparatus according to any one of claims 6 to 10, **characterized in that** the first connection means (207) include a first connection interface (2071), located in a bottom (1116) of the cavity (211), the second means (107) include a second connection interface (1071), located at an upper end (1110) of the protruding portion (111), the second connection interface (1071) being connected to the first connection interface (2071) when the protruding portion (111) is inserted into the cavity (211) in the insertion direction (D) to be in the engaged position.

12. The apparatus according to any one of the preceding claims, **characterized in that** at least one sensor (2061, 2062) for measuring the presence of air flow facing each upper opening (202, 2021, 2022) and at least one other sensor (2063) for measuring the presence of air flow facing each rear opening (203) are provided.

13. The apparatus according to any one of the preceding claims, **characterized in that** there is provided as an air flow presence sensor (206) at least one bare thermistor conductor (2060), extending between two ends connected to an electrical circuit (2065) passing a heating current through the bare thermistor conductor (2060),
the bare thermistor conductor (2060) causing, in response to the air flow passing thereon, a variation in the electrical resistance of the bare thermistor conductor (2060) between its ends (2060a, 2060b), capable of being detected by detection means of the electrical circuit (2065).

14. The apparatus according to claim 13, **characterized in that** the electric circuit (2065) includes bias means for imposing a constant prescribed electric current in the bare thermistor conductor (2060) and means for detecting the electric voltage present between the two ends (2060a, 2060b) of the bare thermistor conductor (2060).

15. The apparatus according to claim 13, **characterized in that** the electrical circuit (2065) includes bias means for imposing a constant prescribed electrical voltage between the two ends (2060a, 2060b) of the bare thermistor conductor (2060) and means for detecting the electric current passing through the bare thermistor conductor (2060).

16. The apparatus according to any one of the preceding claims, **characterized in that** at least one sound sensor (2064), positioned facing the rear opening (203), is provided as an air flow presence sensor (206).

17. The apparatus according to any one of the preceding claims, **characterized in that** it includes a part (500) for filtering air flows, which is removable and intended to be fastened against the first outer wall (201).
